# FASCICULE DE BREVET EUROPEEN

(11) **EP 1 412 386 B1**
(45) Date de publication et mention de la délivrance du brevet: **30.06.2010**
(21) Numéro de dépôt: 02762548.2
(22) Date de dépôt: 26.07.2002
(51) Int. Cl.: C07K 14/705, G01N 33/68, C12Q 1/02

(54) **UTILISATION DE FRAGMENTS PEPTIDIQUES DE LA SOUS-UNITE alpha-1 DES CANAUX CALCIQUES, COMPORTANT LE CAS ECHEANT DES MUTATIONS, POUR LE CRIBLAGE DE MOLECULES D'INTERET THERAPEUTIQUE**
VERWENDUNG VON PEPTIDFRAGMENTEN AUS DER ALPHA-1 UNTEREINHEIT VON KALZIUMKANÄLEN, DIE MUTATIONEN ENTHALTEN KÖNNEN, FÜR DAS SCREENING VON MOLEKÜLEN MIT THERAPEUTISCHEM POTENTIAL
USE OF PEPTIDE FRAGMENTS OF THE CALCIUM CHANNEL alpha-1 SUBUNIT, OPTIONALLY COMPRISING MUTATIONS, FOR SCREENING MOLECULES OF THERAPEUTIC INTEREST

(30) Priorité: 27.07.2001 FR 0110117
(43) Date de publication de la demande: 28.04.2004
(73) Titulaire: CENTRE NATIONAL DE LA RECHERCHE SCIENTIFIQUE, 75794 Paris Cedex 16 (FR)
(72) Inventeur: SABATIER, Jean-Marc, F-13790 Rousset (FR); DE WAARD, Michel CEA-INSERM-EMI9931 Lab.Canaux Ion. et Signalisation, F-38054 Grenoble Cedex 09 (FR)
(74) Mandataire: Grosset-Fournier, Chantal Catherine
(86) Numéro de dépôt international: PCT/FR2002/002679
(87) Numéro de publication internationale: WO 2003/011906

(56) Documents cités:
- US-A- 5 876 958
- BICHET DELPHINE ET AL: "Reversibility of the Ca2+ channel alpha1-beta subunit interaction." BIOCHEMICAL AND BIOPHYSICAL RESEARCH COMMUNICATIONS, vol. 277, no. 3, 2 novembre 2000 (2000-11-02), pages 729-735, XP002198858 ISSN: 0006-291X
- HERING STEFFEN ET AL: "Molecular determinants of inactivation in voltage-gated Ca2+ channels." JOURNAL OF PHYSIOLOGY (CAMBRIDGE), vol. 528, no. 2, 15 octobre 2000 (2000-10-15), pages 237-249, XP001064791 ISSN: 0022-3751
- HERLITZE STEFAN ET AL: "Molecular determinants of inactivation and G protein modulation in the intracellular loop connecting domains I and II of the calcium channel alpha-1A subunit." PROCEEDINGS OF THE NATIONAL ACADEMY OF SCIENCES OF THE UNITED STATES, vol. 94, no. 4, 1997, pages 1512-1516, XP002228874 1997 ISSN: 0027-8424
- M. PRAGNELL ET AL: "Calcium channel beta-subunit binds to a conserved motif in the I-II cytoplasmic linker of the alpha1-subunit" NATURE., vol. 368, 3 mars 1994 (1994-03-03), pages 67-70, XP002198859 MACMILLAN JOURNALS LTD. LONDON., GB ISSN: 0028-0836 cité dans la demande
- BICHET DELPHINE ET AL: "The I-II loop of the Ca2+ channel alpha1 subunit contains an endoplasmic reticulum retention signal antagonized by the beta subunit." NEURON., vol. 25, no. 1, janvier 2000 (2000-01), pages 177-190, XP002198861 ISSN: 0896-6273
- DE WAARD MICHEL ET AL: "IDENTIFICATION OF CRITICAL AMINO ACIDS INVOLVED IN ALPHA-1-BETA INTERACTION IN VOLTAGE-DEPENDENT CA-2+ CHANNELS" FEBS LETTERS, ELSEVIER SCIENCE PUBLISHERS, AMSTERDAM, NL, vol. 380, no. 3, 1996, pages 272-276, XP002155228 ISSN: 0014-5793
- DE WAARD MICHEL ET AL: "Properties of the alpha-1-beta Anchoring Site in Voltage-dependent Ca-2+ Channels." JOURNAL OF BIOLOGICAL CHEMISTRY, vol. 270, no. 20, 1995, pages 12056-12064, XP002198860 ISSN: 0021-9258
- HOFMANN F ET AL: "Voltage-dependent calcium channels: From structure to function." REVIEWS OF PHYSIOLOGY BIOCHEMISTRY AND PHARMACOLOGY, vol. 139, 1999, pages 33-87, XP001076646 1999 Springer-Verlag;Springer-Verlag New York, Inc. Heidelberger Platz 3, D-1000 Berlin, Germany; 175 Fifth Avenue, New York, New York 10010, USA ISBN: 3-540-65694-4
- GEIB SANDRINE ET AL: "The interaction between the I-II loop and the III-IV loop of Cav2.1 contributes to voltage-dependent inactivation in a beta-dependent manner." JOURNAL OF BIOLOGICAL CHEMISTRY, vol. 277, no. 12, 22 mars 2002 (2002-03-22), pages 10003-10013, XP002198862 March 22, 2002 ISSN: 0021-9258
- FATHALLAH MOHAMED ET AL: "Modelling of the III-IV loop, a domain involved in calcium channel Cav2.1 inactivation, highlights a structural homology with the gamma subunit of G proteins." EUROPEAN JOURNAL OF NEUROSCIENCE, vol. 16, no. 2, juillet 2002 (2002-07), pages 219-228, XP002228875 July, 2002 ISSN: 0953-816X

### SEQUENCE LISTING

<110> CENTRE NATIONAL DE LA RECHERCHE SCIENTIFIQUE
<120> UTILISATION DE FRAGMENTS PEPTIDIQUES DE LA SOUS-UNITE α-1 DES CANAUX CALCIQUES, COMPORTANT LE CAS ECHEANT DES MUTATIONS, POUR LE CRIBLAGE DE MOLECULES D'INTERET THERAPEUTIQUE
<130> IFB 01AE CNR CANA
<140> FR0110117
<141> 2001-07-27
<160> 15
<170> PatentIn version 3.1
<210> 1
   <211> 384
   <212> DNA
   <213> lapin
<220>
   <221> CDS
   <222> (1) .. (384)
   <223>
<400> 1
   tca ggg gag ttt gcc aaa gaa agg gag cgg gtg gag aac cgg cgc gca      48
   Ser Gly Glu Phe Ala Lys Glu Arg Glu Arg Val Glu Asn Arg Arg Ala
   1               5                   10                  15
   ttc ctg aag ctg cgg cgg cag cag cag att gaa cgc gag ctc aac ggg      96
   Phe Leu Lys Leu Arg Arg Gln Gln Gln Ile Glu Arg Glu Leu Asn Gly
               20                  25                  30
   tac atg gag tgg atc tca aaa gca gaa gag gtg atc ctc gca gag gac      144
   Tyr Met Glu Trp Ile Ser Lys Ala Glu Glu Val Ile Leu Ala Glu Asp
           35                  40                  45
   gag acc gac gtg gag cag aga cat ccc ttt gat gga gct ctg cgg aga      192
   Glu Thr Asp Val Glu Gln Arg His Pro Phe Asp Gly Ala Leu Arg Arg
       50                  55                  60
   gcc act atc aag aag agc aag acg gac ctg ctc cac cca gag gag gcg      240
   Ala Thr Ile Lys Lys Ser Lys Thr Asp Leu Leu His Pro Glu Glu Ala
   65                  70                  75                  80
   gag gat cag ctg gcc gac atc gcc tcc gtg ggg tct ccc ttt gcc cga      288
   Glu Asp Gln Leu Ala Asp Ile Ala Ser Val Gly Ser Pro Phe Ala Arg
                   85                  90                  95
   gcc agc att aaa agt gcc aag ctg gag aac tcg agt ttt ttc cac aaa      336
   Ala Ser Ile Lys Ser Ala Lys Leu Glu Asn Ser Ser Phe Phe His Lys
               100                 105                 110
   aaa gag agg aga atg cgt ttc tac atc cgt cgc atg gtc aaa act cag      384
   Lys Glu Arg Arg Met Arg Phe Tyr Ile Arg Arg Met Val Lys Thr Gln
           115                 120                 125
<210> 2
   <211> 128
   <212> PRT
   <213> lapin
<400> 2
   Ser Gly Glu Phe Ala Lys Glu Arg Glu Arg Val Glu Asn Arg Arg Ala
   1               5                   10                  15
   Phe Leu Lys Leu Arg Arg Gln Gln Gln Ile Glu Arg Glu Leu Asn Gly
               20                  25                  30
   Tyr Met Glu Trp Ile Ser Lys Ala Glu Glu Val Ile Leu Ala Glu Asp
           35                  40                  45
   Glu Thr Asp Val Glu Gln Arg His Pro Phe Asp Gly Ala Leu Arg Arg
       50                  55                  60
   Ala Thr Ile Lys Lys Ser Lys Thr Asp Leu Leu His Pro Glu Glu Ala
   65                  70                  75                  80
   Glu Asp Gln Leu Ala Asp Ile Ala Ser Val Gly Ser Pro Phe Ala Arg
                   85                  90                  95
   Ala Ser Ile Lys Ser Ala Lys Leu Glu Asn Ser Ser Phe Phe His Lys
               100                 105                 110
   Lys Glu Arg Arg Met Arg Phe Tyr Ile Arg Arg Met Val Lys Thr Gln
           115                 120                 125
<210> 3
   <211> 384
   <212> DNA
   <213> Homo sapiens
<220>
   <221> CDS
   <222> (1)..(384)
   <223>
<400> 3
   tca ggg gag ttt gcc aaa gaa agg gaa cgg gtg gag aac cgg cgg gct      48
   Ser Gly Glu Phe Ala Lys Glu Arg Glu Arg Val Glu Asn Arg Arg Ala
   1               5                   10                  15
   ttt ctg aag ctg agg cgg caa caa cag att gaa cgt gag ctc aat ggg      96
   Phe Leu Lys Leu Arg Arg Gln Gln Gln Ile Glu Arg Glu Leu Asn Gly
               20                  25                  30
   tac atg gaa tgg atc tca aaa gca gaa gag gtg atc ctc gcc gag gat     144
   Tyr Met Glu Trp Ile Ser Lys Ala Glu Glu Val Ile Leu Ala Glu Asp
           35                  40                  45
   gaa act gac ggg gag cag agg cat ccc ttt gat gga gct ctg cgg aga     192
   Glu Thr Asp Gly Glu Gln Arg His Pro Phe Asp Gly Ala Leu Arg Arg
       50                  55                  60
   acc acc ata aag aaa agc aag aca gat ttg ctc aac ccc gaa gag gct     240
   Thr Thr Ile Lys Lys Ser Lys Thr Asp Leu Leu Asn Pro Glu Glu Ala
   65                  70                  75                  80
   gag gat cag ctg gct gat ata gcc tct gtg ggt tct ccc ttc gcc cga     288
   Glu Asp Gln Leu Ala Asp Ile Ala Ser Val Gly Ser Pro Phe Ala Arg
                   85                  90                  95
   gcc agc att aaa agt gcc aag ctg gag aac tcg acc ttt ttt cac aaa     336
   Ala Ser Ile Lys Ser Ala Lys Leu Glu Asn Ser Thr Phe Phe His Lys
               100                 105                 110
   aag gag agg agg atg cgt ttc tac atc cgc cgc atg gtc aaa act cag     384
   Lys Glu Arg Arg Met Arg Phe Tyr Ile Arg Arg Met Val Lys Thr Gln
           115                 120                 125
<210> 4
   <211> 128
   <212> PRT
   <213> Homo sapiens
<400> 4
   Ser Gly Glu Phe Ala Lys Glu Arg Glu Arg Val Glu Asn Arg Arg Ala
   1               5                   10                  15
   Phe Leu Lys Leu Arg Arg Gln Gln Gln Ile Glu Arg Glu Leu Asn Gly
               20                  25                  30
   Tyr Met Glu Trp Ile Ser Lys Ala Glu Glu Val Ile Leu Ala Glu Asp
           35                  40                  45
   Glu Thr Asp Gly Glu Gln Arg His Pro Phe Asp Gly Ala Leu Arg Arg
   50 55 60
   Thr Thr Ile Lys Lys Ser Lys Thr Asp Leu Leu Asn Pro Glu Glu Ala
   65 70 75 80
   Glu Asp Gln Leu Ala Asp Ile Ala Ser Val Gly Ser Pro Phe Ala Arg
   85 90 95
   Ala Ser Ile Lys Ser Ala Lys Leu Glu Asn Ser Thr Phe Phe His Lys
   100 105 110
   Lys Glu Arg Arg Met Arg Phe Tyr Ile Arg Arg Met Val Lys Thr Gln
   115 120 125
<210> 5
   <211> 33
   <212> PRT
   <213> Homo sapiens
<400> 5
   Leu Asp Phe Ala Cys Asp Ile Tyr Ile Trp Ala Pro Leu Ala Gly Thr
   1 5 10 15
   Cys Gly Val Leu Leu Leu Ser Leu Val Ile Thr Leu Tyr Cys Asn His
   20 25 30
   Arg
<210> 6
   <211> 165
   <212> DNA
   <213> Homo sapiens
<220>
   <221> CDS
   <222> (1)..(165)
   <223>
<400> 6
   atc acc ttc cag gag caa ggg gac aag atg atg gag gaa tac agc ctg        48
   Ile Thr Phe Gln Glu Gln Gly Asp Lys Met Met Glu Glu Tyr Ser Leu
   1               5                   10                  15
   gag aaa aat gag agg gcc tgc att gat ttc gcc atc agc gcc aag ccg        96
   Glu Lys Asn Glu Arg Ala Cys Ile Asp Phe Ala Ile Ser Ala Lys Pro
               20                  25                  30
   ctg acc cga cac atg ccg cag aac aag cag agc ttc cag tac cgc atg       144
   Leu Thr Arg His Met Pro Gln Asn Lys Gln Ser Phe Gln Tyr Arg Met
           35                  40                  45
   tgg cag ttc gtg gtg tct ccg                                           165
   Trp Gln Phe Val Val Ser Pro
       50                  55
<210> 7
   <211> 55
   <212> PRT
   <213> Homo sapiens
<400> 7
   Ile Thr Phe Gln Glu Gln Gly Asp Lys Met Met Glu Glu Tyr Ser Leu
   1               5                   10                  15
   Glu Lys Asn Glu Arg Ala Cys Ile Asp Phe Ala Ile Ser Ala Lys Pro
               20                  25                  30
   Leu Thr Arg His Met Pro Gln Asn Lys Gln Ser Phe Gln Tyr Arg Met
           35                  40                  45
   Trp Gln Phe Val Val Ser Pro
       50                  55
<210> 8
   <211> 165
   <212> DNA
   <213> lapin
<400> 8
   atcaccttcc aggagcaggg cgacaagatg atggaggagt acagcttgga gaaaaacgag     60
   agggcctgca tcgacttcgc catcagtgcc aagccgctga ccaggcacat gccccagaac    120
   aagcagagct tccagtaccg catgtggcag ttcgtggtgt ccccg                    165
<210> 9
   <211> 128
   <212> PRT
   <213> lapin
<220>
   <221> MISC_FEATURE
   <222> (24)..(24)
   <223> X représente Q, ou une alanine, ou autre acide aminé naturel ou non i
<220>
   <221> MISC_FEATURE
   <222> (36).. (36)
   <223> X représente W, ou une alanine, ou autre acide aminé naturel ou non
<220>
   <221> MISC_FEATURE
   <222> (37) .. (37)
   <223> X représente I, ou une alanine, ou autre acide aminé naturel ou non
<220>
   <221> MISC_FEATURE
   <222> (39)..(39)
   <223> X représente K, ou une alanine, ou autre acide aminé naturel ou non
<220>
   <221> MISC_FEATURE
   <222> (68)..(69)
   <223> X représente K, ou une alanine, ou autre acide aminé naturel ou non
<400> 9
   Ser Gly Glu Phe Ala Lys Glu Arg Glu Arg Val Glu Asn Arg Arg Ala
   1               5                   10                  15
   Phe Leu Lys Leu Arg Arg Gln Xaa Gln Ile Glu Arg Glu Leu Asn Gly
               20                  25                  30
   Tyr Met Glu Xaa Xaa Ser Xaa Ala Glu Glu Val Ile Leu Ala Glu Asp
           35                  40                  45
   Glu Thr Asp Val Glu Gln Arg His Pro Phe Asp Gly Ala Leu Arg Arg
       50                  55                  60
   Ala Thr Ile Xaa Xaa Ser Lys Thr Asp Leu Leu His Pro Glu Glu Ala
   65                  70                  75                  80
   Glu Asp Gln Leu Ala Asp Ile Ala Ser Val Gly Ser Pro Phe Ala Arg
                   85                  90                  95
   Ala Ser Ile Lys Ser Ala Lys Leu Glu Asn Ser Ser Phe Phe His Lys
               100                 105                 110
   Lys Glu Arg Arg Met Arg Phe Tyr Ile Arg Arg Met Val Lys Thr Gln
           115                 120                 125
<210> 10
   <211> 128
   <212> PRT
   <213> Homo sapiens
<220>
   <221> MISC_FEATURE
   <222> (24)..(24)
   <223> X représente Q, ou une alanine, ou autre acide aminé naturel ou non
<220>
   <221> MISC_FEATURE
   <222> (36)..(36)
   <223> X représente W, ou une alanine, ou autre acide aminé naturel ou non
<220>
   <221> MISC_FEATURE
   <222> (37)..(37)
   <223> X représente I, ou une alanine, ou autre acide aminé naturel ou non
<220>
   <221> MISC_FEATURE
   <222> (39)..(39)
   <223> X représente K, ou une alanine, ou autre acide aminé naturel ou non
<220>
   <221> MISC_FEATURE
   <222> (68)..(69)
   <223> X représente K, ou une alanine, ou autre acide aminé naturel ou non
<400> 10 :
   Ser Gly Glu Phe Ala Lys Glu Arg Glu Arg Val Glu Asn Arg Arg Ala
   1 5 10 15
   Phe Leu Lys Leu Arg Arg Gln Xaa Gln Ile Glu Arg Glu Leu Asn Gly
   20 25 30
   Tyr Met Glu Xaa Xaa Ser Xaa Ala Glu Glu Val Ile Leu Ala Glu Asp
   35 40 45
   Glu Thr Asp Gly Glu Gln Arg His Pro Phe Asp Gly Ala Leu Arg Arg
   50 55 60
   Thr Thr Ile Xaa Xaa Ser Lys Thr Asp Leu Leu Asn Pro Glu Glu Ala
   65 70 75 80
   Glu Asp Gln Leu Ala Asp Ile Ala Ser Val Gly Ser Pro Phe Ala Arg
   85 90 95
   Ala Ser Ile Lys Ser Ala Lys Leu Glu Asn Ser Thr Phe Phe His Lys
   100 105 110
   Lys Glu Arg Arg Met Arg Phe Tyr Ile Arg Arg Met Val Lys Thr Gln
   115 120 125
<210> 11
   <211> 128
   <212> PRT
   <213> lapin
<220>
   <221> MISC_FEATURE
   <222> (28)..(28)
   <223> X représente R, ou une alanine, ou autre acide aminé naturel ou non
<220>
   <221> MISC_FEATURE
   <222> (63)..(64)
   <223> X représente R, ou une alanine, ou autre acide aminé naturel ou non
<400> 11
   Ser Gly Glu Phe Ala Lys Glu Arg Glu Arg Val Glu Asn Arg Arg Ala
   1               5                   10                  15
   Phe Leu Lys Leu Arg Arg Gln Gln Gln Ile Glu Xaa Glu Leu Asn Gly
               20                  25                  30
   Tyr Met Glu Trp Ile Ser Lys Ala Glu Glu Val Ile Leu Ala Glu Asp
           35                  40                  45
   Glu Thr Asp Val Glu Gln Arg His Pro Phe Asp Gly Ala Leu Xaa Xaa
       50                  55                  60
   Ala Thr Ile Lys Lys Ser Lys Thr Asp Leu Leu His Pro Glu Glu Ala
   65                  70                  75                  80
   Glu Asp Gln Leu Ala Asp Ile Ala Ser Val Gly Ser Pro Phe Ala Arg
                   85                  90                  95
   Ala Ser Ile Lys Ser Ala Lys Leu Glu Asn Ser Ser Phe Phe His Lys
               100                 105                 110
   Lys Glu Arg Arg Met Arg Phe Tyr Ile Arg Arg Met Val Lys Thr Gln
           115                 120                 125
<210> 12
   <211> 128
   <212> PRT
   <213> Homo sapiens
<220>
   <221> MISC_FEATURE
   <222> (28)..(28)
   <223> X représente R, ou une alanine, ou autre acide aminé naturel ou non
<220>
   <221> MISC_FEATURE
   <222> (63)..(64)
   <223> X représente R, ou une alanine, ou autre acide aminé naturel ou non
<400> 12
   Ser Gly Glu Phe Ala Lys Glu Arg Glu Arg Val Glu Asn Arg Arg Ala
   1 5 10 15
   Phe Leu Lys Leu Arg Arg Gln Gln Gln Ile Glu Xaa Glu Leu Asn Gly
   20 25 30
   Tyr Met Glu Trp Ile Ser Lys Ala Glu Glu Val Ile Leu Ala Glu Asp
   35 40 45
   Glu Thr Asp Gly Glu Gln Arg His Pro Phe Asp Gly Ala Leu Xaa Xaa
   50 55 60
   Thr Thr Ile Lys Lys Ser Lys Thr Asp Leu Leu Asn Pro Glu Glu Ala
   65 70 75 80
   Glu Asp Gln Leu Ala Asp Ile Ala Ser Val Gly Ser Pro Phe Ala Arg
   85 90 95
   Ala Ser Ile Lys Ser Ala Lys Leu Glu Asn Ser Thr Phe Phe His Lys
   100 105 110
   Lys Glu Arg Arg Met Arg Phe Tyr Ile Arg Arg Met Val Lys Thr Gln
   115 120 125
<210> 13
   <211> 128
   <212> PRT
   <213> lapin
<220>
   <221> MISC_FEATURE
   <222> (28)..(28)
   <223> X représente R, ou une alanine, ou autre acide aminé naturel ou non
<220>
   <221> MISC_FEATURE
   <222> (29) .. (29)
   <223> X représente E, ou une alanine, ou autre acide aminé naturel ou non
<400> 13
   Ser Gly Glu Phe Ala Lys Glu Arg Glu Arg Val Glu Asn Arg Arg Ala
   1 5 10 15
   Phe Leu Lys Leu Arg Arg Gln Gln Gln Ile Glu Xaa Xaa Leu Asn Gly
   20 25 30
   Tyr Met Glu Trp Ile Ser Lys Ala Glu Glu Val Ile Leu Ala Glu Asp
   35 40 45
   Glu Thr Asp Val Glu Gln Arg His Pro Phe Asp Gly Ala Leu Arg Arg
   50 55 60
   Ala Thr Ile Lys Lys Ser Lys Thr Asp Leu Leu His Pro Glu Glu Ala
   65 70 75 80
   Glu Asp Gln Leu Ala Asp Ile Ala Ser Val Gly Ser Pro Phe Ala Arg
   85 90 95
   Ala Ser Ile Lys Ser Ala Lys Leu Glu Asn Ser Ser Phe Phe His Lys
   100 105 110
   Lys Glu Arg Arg Met Arg Phe Tyr Ile Arg Arg Met Val Lys Thr Gln
   115 120 125
<210> 14
   <211> 128
   <212> PRT
   <213> Homo sapiens
<220>
   <221> MISC_FEATURE
   <222> (28)..(28)
   <223> X représente R, ou une alanine, ou autre acide aminé naturel ou non
<220>
   <221> MISC_FEATURE
   <222> (29) .. (29)
   <223> X représente E, ou une alanine, ou autre acide aminé naturel ou non
<400> 14
   Ser Gly Glu Phe Ala Lys Glu Arg Glu Arg Val Glu Asn Arg Arg Ala
   1 5 10 15
   Phe Leu Lys Leu Arg Arg Gln Gln Gln Ile Glu Xaa Xaa Leu Asn Gly
               20                  25                  30
   Tyr Met Glu Trp Ile Ser Lys Ala Glu Glu Val Ile Leu Ala Glu Asp
           35                  40                  45
   Glu Thr Asp Gly Glu Gln Arg His Pro Phe Asp Gly Ala Leu Arg Arg
       50                  55                  60
   Thr Thr Ile Lys Lys Ser Lys Thr Asp Leu Leu Asn Pro Glu Glu Ala
   65                  70                  75                  80
   Glu Asp Gln Leu Ala Asp Ile Ala Ser Val Gly Ser Pro Phe Ala Arg
                   85                  90                  95
   Ala Ser Ile Lys Ser Ala Lys Leu Glu Asn Ser Thr Phe Phe His Lys
               100                 105                 110
   Lys Glu Arg Arg Met Arg Phe Tyr Ile Arg Arg Met Val Lys Thr Gln
           115                 120                 125
<210> 15
   <211> 17
   <212> PRT
   <213> lapin
<400> 15

## Description

La présente invention a pour objet l'utilisation de fragments peptidiques de la sous-unité α-1 des canaux calciques de mammifères, de séquences dérivées par mutation desdits fragments, ou encore de cellules transformés par des séquences codant pour lesdits fragment ou séquences dérivées, pour le criblage de molécules d'intérêt thérapeutique.

Des canaux calcium haut-seuils dépendant du potentiel comprennent la sous-unité α-1 et la sous-unité β. La sous-unité α-1 et la sous-unité β interagissent par deux régions cytoplasmiques nommées comme AID (Alpha interaction Domain) et BID (Beta Interaction Demain) (De Waard et al., FEBS Letters 380 (1996), 272-276).

L'importance de la boucle I-II dans la régulation de l'activité du canal est connu depuis 1994, date à laquelle le site d'ancrage de la sous-unité β auxiliaire fut identifié (Pragnell M. et al., (1994), « Calcium channel beta-subunit binds to a conserved motif in the I-II cytoplasmic linker of the alpha 1-subunit »Nature 368, 67-70 ; De Waard et al., The Journal of Biological Chemistry, Vol. 270, No. 20, Issue of May 19, pp. 12056-12064). Cette sous-unité β produit un nombre impressionnant de modifications fonctionnelles des canaux calciques.

La boucle cytoplasmique I-II de la sous-unité α-1 des canaux calcium haut-seuils dépendants du potentiel relie entre elles le premier et le second des quatre domaines hydrophobes. La boucle I-II de la sous-unité α-1 des canaux calciques contient un signal de rétention de réticulum endoplasmique qui restreint sévèrement l'incorporation de la sous-unité α-1 dans la membrane plasmatique. En revanche, la sous-unité β renverse cette inhibition imposée par le signal de rétention (Bichet et al., Neuron, Vol., 177-190, January, 2000). La boucle I-II joue un rôle essentiel dans 1) la régulation de l'activité du canal (propriétés d'activation et d'inactivation) (Hering et al., J. Physiol. 2000 ; 528 ; 237-249), 2) le niveau d'expression membranaire du canal (contrôle du niveau de rétention au sein du réticulum endoplasmique), 3) la régulation par les protéines exogènes (site de fixation du complexe Gβγ des protéines G) (Herlize et al., Proc.Natl. Acad.Sci. USA Vol. 94 ; 1512-1516) et 4) la régulation par les sous-unité β des canaux calciques (site d'ancrage de la sous-unité β et premier site de régulation) (Bichet et al., Biochemical and Biophysical Research Communications 277, 729-735 (2000)). D'après les résultats expérimentaux obtenus par les Inventeurs, il apparaît particulièrement intéressant d'utiliser la boucle I-II des canaux calciques pour la compréhension des rôles cellulaires de ces canaux calciques, pour l'inactivation neuronale, ou encore comme une cible pharmacologique pour la modulation de l'activité des canaux calciques.

En effet l'invention découle de la mise en évidence par les Inventeurs du fait que l'expression exogène de la boucle I-II, lorsqu'elle est couplée à un segment transmembranaire, inhibe de manière substantielle l'expression membranaire de canaux calciques natifs. Ce résultat permet de réduire l'expression de canaux calciques dans un tissu donné afin d'appréhender la fonction cellulaire de ces canaux.

Les Inventeurs ont démontré que la boucle I-II est une boucle moléculaire organisatrice. Elle est couplée à d'autres boucles cytoplasmiques du canal calcique (séquences amino et carboxy-terminales, boucles II-III et III-IV). Ces interactions intra-moléculaires ont pour fonction le contrôle de l'inactivation. Par exemple, l'interaction entre boucle I-II et boucle III-IV du canal calcium de type P/Q a joué un rôle primordial dans le contrôle de la cinétique d'inactivation du canal. De façon spectaculaire, ces interactions sont partiellement ou totalement interrompues par l'interaction de la boucle I-II avec la sous-unité β. En d'autres termes, la sous-unité β des canaux calcium entre en compétition avec les interactions intramoléculaires de la boucle I-II. Avec de tels résultats, on peut s'attendre à ce que le complexe Gβγ, qui lui aussi se fixe sur la boucle I-II, puisse aussi entrer en compétition avec ces interactions moléculaires. Ce serait par le biais de ces compétitions que le complexe Gβγ provoquerait la régulation du canal calcique (diminution de l'amplitude du courant calcique et ralentissement de la cinétique d'activation).

Ainsi, un des principaux buts de la présente invention est de développer un test de criblage visant à identifier des molécules capables de perturber les interactions intramoléculaires et l'activité ionique de la sous-unité α₁ des canaux calciques à hauts-seuils, lesdites molécules étant susceptibles d'être utilisées notamment dans le cadre du traitement de l'ischémie cérébrale ou de la neurodégénérescence. La méthode générale d'un criblage de molécules intéressantes a été décrite dans le brevet US 5, 876, 958.

Les Inventeurs ont mis en oeuvre une méthode de criblage des molécules capable de perturber l'activité ionique de la sous-unité α₁ des canaux calciques à hauts-seuils par l'utilisation
- de fragments peptidiques de la sous-unité α₁ des canaux calciques de mammifères, lesdits fragments correspondant à la boucle I-II et/ou à la boucle III-IV de ladite sous-unité α1, ou correspondant à une séquence peptidique dérivée de cette boucle I-II ou III-IV, notamment par substitution, et/ou délétion, et/ou addition d'un ou plusieurs acides aminés, ou correspondant à une partie peptidique de ladite boucle I-II, ou III-IV, ou d'une séquence dérivée de cette dernière, notamment à une partie peptidique d'au moins environ 5 acide aminés, ladite séquence dérivée et ladite partie de la boucle I-II ayant la propriété de ladite boucle I-II de se lier à la sous-unité β et à la boucle III-IV desdits canaux calciques, ladite séquence dérivée et ladite partie de la boucle III-IV ayant la propriété de ladite boucle III-IV de se lier à ladite boucle I-II,
- ou de séquences peptidiques mutées, dérivées par mutation d'un ou plusieurs acides aminés desdits fragments peptidiques susmentionnés de la boucle I-II de la sous-unité α1 des canaux calciques dans la mesure où la ou les mutations en question affectent des acides aminés essentiels dans le cadre de l'expression des canaux calciques à la surface membranaire, et/ou de séquences peptidiques dérivées par mutation d'un ou plusieurs acides aminés desdits fragments peptidiques susmentionnés de la boucle III-IV de la sous-unité α1 des canaux calciques, dans la mesure où la ou les mutations en question affectent des acides aminés essentiels dans le cadre de l'inactivation des canaux calciques,
- ou de cellules transformées par des séquences nucléotidiques codant pour les fragments peptidiqùes susmentionnés de la boucle I-II et/ou de la boucle III-IV de la sous-unité α1 des canaux calciques, ou codant pour les séquences peptidiques mutées susmentionnées,
pour la mise en oeuvre de procédés de criblage :
- de molécules restaurant le nombre de canaux calciques à la normale dans les membranes cellulaires où ce nombre a anormalement diminué, à savoir du criblage de molécules β like susceptibles d'être utilisées dans le cadre du traitement de pathologies liées à une diminution anormale du nombre de canaux calciques telles que l'épilepsie, ou les dégénérescences neuronales,
- et/ou de molécules augmentant le nombre de canaux calciques dans les membranes cellulaires, à savoir du criblage de molécules β like susceptibles d'être utilisées dans le cadre du traitement de pathologies contre lesquelles une augmentation du nombre de canaux calciques à la membrane plasmique aurait un effet bénéfique, telle que la maladie de Parkinson, les diabètes insulino-dépendants, ou le syndrome myasthénique de Lambert-Eaton,,
- et/ou de molécules restaurant le nombre de canaux calciques à la normale dans les membranes cellulaires où ce nombre a anormalement augmenté, à savoir du criblage de molécules susceptibles d'être utilisées dans le cadre du traitement de pathologies liées à une augmentation anormale du nombre de canaux calciques telle que l'hypertrophie cardiaque,
- et/ou de molécules réduisant le nombre de canaux calciques dans les membranes cellulaires, à savoir du criblage de molécules susceptibles d'être utilisées dans le cadre du traitement de pathologies contre lesquelles une diminution du nombre de canaux calciques à la membrane plasmique aurait un effet bénéfique, telle que l'épilepsie, l'hypertension, l'angine de poitrine, ou l'ischémie cérébrale,
- et/ou de molécules régulant l'état d'inactivation des canaux calciques neuronaux impliqués dans la libération des neurotransmetteurs, à savoir du criblage de molécules susceptibles d'être utilisées dans le cadre de la stimulation ou de l'inhibition de la communication neuronale, notamment dans le cadre du traitement de pathologies contre lesquelles une régulation de l'état d'inactivation des canaux calciques neuronaux impliqués dans la libération des neurotransmetteurs aurait un effet bénéfique, telles que les épilepsies, les ataxies, les migraines, la maladie de Parkinson, ou les ischémies cérébrales.

Dans les études précédentes, les Inventeurs ont utilisé des fragments peptidiques correspondant à la boucle I-II de la sous-unité α1, ou à une séquence peptidique dérivée, ou à une partie de cette boucle I-II, ou de cellules transformées par des séquences nucléotidiques codant pour lesdits fragments, tels que définis ci-dessus, pour la mise en oeuvre de procédés de criblage :
- de molécules restaurant le nombre de canaux calciques à la normale dans les membranes cellulaires où ce nombre a anormalement diminué, telles que définies ci-dessus,
- et/ou de molécules augmentant le nombre de canaux calciques dans les membranes cellulaires, telles que définies ci-dessus.

Les Inventeurs ont utilisé la séquence peptidique correspondant à la boucle I-II de la sous-unité Cav2.1 des canaux calciques de cellules neuronales de lapin, ladite séquence correspondant à la séquence SEQ ID NO : 2 suivante :
- ou de la séquence peptidique correspondant à la boucle I-II de la sous-unité Cav2.1 des canaux calciques de cellules neuronales humaines, ladite séquence correspondant à la séquence SEQ ID NO : 4 suivante :
- ou de fragments des séquences SEQ ID NO : 2 et SEQ ID NO : 4 susmentionnées, comprenant au moins la séquence SEQ ID NO : 15 suivante : QQIERELNGYMEWISKAE,
- ou de cellules transformées avec les séquences nucléotidiques SEQ ID NO : 1 et SEQ ID NO : 3 suivantes codant respectivement pour les séquences peptidiques SEQ ID NO : 2 et SEQ ID NO : 4 susmentionnées, ou transformées avec la séquence nucléotidique comprise dans les séquences SEQ ID NO : 1 et 3 et codant pour la séquence SEQ ID NO : 15 susmentionnée :

De préférence, dans le cas de l'utilisation susmentionnée de fragments peptidiques de la sous-unité α1 ayant une taille supérieure à environs 5 acides aminés, lesdits fragments sont fusionnés du côté N-terminal à une séquence peptidique transmembranaire, à savoir une séquence peptidique ayant pour effet de maintenir lesdits fragments peptidiques dans la membrane cellulaire, telle que la séquence transmembranaire de la chaîne α du récepteur CD8 humain contenue dans la séquence SEQ ID NO : 5 suivante :
LDFACDIYIWAPLAGTCGVLLLSLVITLYCNHR

En variante, les Inventeurs utilisent également de cellules transformées avec une séquence nucléotidique recombinante exogène codant pour une séquence peptidique transmembranaire telle que définie ci-dessus, cette dernière séquence étant située en amont de la séquence codant pour le fragment peptidique susmentionné de la sous-unité α1.

Précédemment, les Inventeurs ont mis en oeuvre un procédé de criblage de molécules restaurant le nombre de canaux calciques à la normale dans les membranes cellulaires où ce nombre a anormalement diminué, telles que définies ci-dessus, et/ou de molécules augmentant le nombre de canaux calciques dans les membranes cellulaires, telles que définies ci-dessus, caractérisé en ce qu'il comprend les étapes suivantes :
- mise en présence de fragments peptidiques de la sous-unité α1 tels que définis ci-dessus, avec des cellules exprimant des canaux calciques pendant un temps suffisant pour que le nombre de canaux calciques diminue de manière significative à la surface desdites cellules, puis avec des molécules à tester,
- ou mise en présence de cellules transformées à l'aide de séquences nucléotidiques codant pour des fragments peptidiques de la sous-unité α1 tels que définis ci-dessus, réduisant ainsi le nombre de canaux calciques à la surface desdites cellules, avec des molécules à tester,
- détection d'une éventuelle augmentation du nombre de canaux calciques à la surface des cellules transformées témoignant de l'effet des molécules testées d'augmentation du nombre de canaux calciques à la surface des membranes cellulaire, notamment par mesures électrophysiologiques, ou à l'aide de sondes fluorescentes ou radioactives.

Les Inventeurs ont particulièrement utilisé les séquences peptidiques dérivées par mutation d'un ou plusieurs acides aminés des fragments peptidiques de la boucle I-II de la sous-unité α1 des canaux calciques, la ou les mutations en question affectant des acides aminés essentiels dans le cadre de l'expression des canaux calciques à la surface membranaire, dans la mesure où leur mutation a pour effet d'augmenter ou de diminuer l'expression à la surface de la membrane plasmique des canaux calciques, pour la mise en oeuvre de procédés de criblage:
- de molécules restaurant le nombre de canaux calciques à la normale dans les membranes cellulaires où ce nombre a anormalement diminué, telles que définies ci-dessus,
- et/ou de molécules augmentant le nombre de canaux calciques dans les membranes cellulaires, telles que définies ci-dessus,
- et/ou de molécules restaurant le nombre de canaux calciques à la normale dans les membranes cellulaires où ce nombre a anormalement augmenté, telles que définies ci-dessus,
- et/ou de molécules réduisant le nombre de canaux calciques dans les membranes cellulaires, telles que définies ci-dessus.

Les Inventeurs ont utilisé dans leurs recherches précédentes plus particulièrement les séquences peptidiques comportant une ou plusieurs mutations ayant pour effet d'augmenter l'expression à la surface de la membrane plasmique des canaux calciques, lesdites séquences peptidiques étant dérivées par mutation d'un au moins des acides aminés situés aux positions 383, 395, 396, 398, 427, et 428 de la sous-unité Cav2.1 des canaux calciques de cellules neuronales de lapin, ou humaines, à savoir des séquences peptidiques correspondant à la séquence SEQ ID NO : 2 ou SEQ ID NO : 4, dans laquelle l'un au moins de Q en position 24, W en position 36, I en position 37, K en position 39, K en position 68, et K en position 69, est substitué par un acide aminé naturel ou non, notamment par une alanine.

Les Inventeurs ont utilisé dans leurs recherches précédentes plus particulièrement encore les séquences peptidiques comportant une ou plusieurs mutations ayant pour effet de diminuer l'expression à la surface de la membrane plasmique des canaux calciques, lesdites séquences peptidiques étant dérivées par mutation d'un au moins des acides aminés situés aux positions 387, 422, et 423 de la sous-unité Cav2.1 des canaux calciques de cellules neuronales de lapin, ou aux positions équivalentes de la sous-unité Cav2.1 humaine, à savoir des séquences peptidiques correspondant à la séquence SEQ ID NO : 2 ou SEQ ID NO : 4, dans laquelle l'un au moins de R en position 28, R en position 63, et R en position 64, est substitué par un acide aminé naturel ou non, notamment par une alanine.

Précédemment, les Inventeurs ont mis en oeuvre un procédé de criblage :
- de molécules restaurant le nombre de canaux calciques à la normale dans les membranes cellulaires où ce nombre a anormalement diminué, telles que définies ci-dessus,
- et/ou de molécules augmentant le nombre de canaux calciques dans les membranes cellulaires, telles que définies ci-dessus,
- et/ou de molécules restaurant le nombre de canaux calciques à la normale dans les membranes cellulaires où ce nombre a anormalement augmenté, telles que définies dans la revendication 1,
- et/ou de molécules réduisant le nombre de canaux calciques dans les membranes cellulaires, telles que définies ci-dessus,
caractérisé en ce qu'il comprend les étapes suivantes :
- mise en présence de séquences peptidiques dérivées de la boucle I-II de la sous-unité α1 tels que définies ci-dessus, avec des molécules à tester déjà sélectionnées pour leur capacité à se lier spécifiquement aux séquences peptidiques non mutées correspondant auxdites boucles I-II,
- sélection des molécules susmentionnées se liant spécifiquement aux boucles I-II et ne se liant pas aux séquences peptidiques dérivées susmentionnées, notamment par l'utilisation de la technique de Biacore (O'Shannessy DJ et al. (1992) « Immobilization chemistries suitable for use in the Biacore surface plasmon resonance detector » Anal Biochem. 205, 132-136) basée sur un principe physique et optique d'interaction moléculaire,
- le cas échéant, mise en présence des molécules sélectionnées à l'étape précédente avec des cellules exprimant des canaux calciques, et observation de l'éventuel effet des molécules sélectionnées sur l'augmentation ou la diminution du nombre de canaux calciques à la surface desdites cellules, notamment par mesures éléctrophysiologiques, ou à l'aide de sondes fluorescentes ou radioactives.

Précédemment, les Inventeurs utilisent les séquences peptidiques dérivées par mutation d'un ou plusieurs acides aminés des fragments peptidiques de la boucle I-II de la sous-unité α1 des canaux calciques, la ou les mutations en question affectant des acides aminés essentiels dans le cadre de l'inactivation des canaux calciques présents à la surface membranaire, dans la mesure où leur mutation a pour effet de moduler l'activité des canaux calciques, pour la mise en oeuvre de procédés de criblage de molécules régulant l'état d'inactivation des canaux calciques neuronaux impliqués dans la libération des neurotransmetteurs, telles que définies ci-dessus.

Dans leurs recherches précédentes, les Inventeurs ont utilisé plus particulièrement les séquences peptidiques comportant une ou plusieurs mutations ayant pour effet d'inactiver les canaux calciques, lesdites séquences peptidiques étant dérivées par mutation d'un au moins des acides aminés situés aux positions 387 et 388, et plus particulièrement par la seule mutation de l'acide aminé situé en position 388, de la sous-unité Cav2.1 des canaux calciques de cellules neuronales de lapin, ou humaines, à savoir des séquences peptidiques correspondant à la séquence SEQ ID NO : 2 ou SEQ ID NO : 4, dans laquelle l'un au moins de R en position 28, et E en position 29, substitué par un acide aminé naturel ou non, notamment R28 est substitué par une alanine ou par E, et E29 est substitué par une alanine.

Dans leurs recherches précédentes, les Inventeurs ont mis en oeuvre un crillage de molécules régulant l'état d'inactivation des canaux calciques neuronaux impliqués dans la libération des neurotransmetteurs, telles que définies ci-dessus, caractérisé en ce qu'il comprend les étapes suivantes :
- mise en présence de séquences peptidiques dérivées de la boucle I-II de la sous-unité α1 tels que définies ci-dessus, avec des molécules à tester déjà sélectionnées pour leur capacité à se lier spécifiquement aux séquences peptidiques non mutées correspondant auxdites boucles I-II,
- sélection des molécules susmentionnées se liant spécifiquement aux boucles I-II et ne se liant pas aux séquences peptidiques dérivées susmentionnées, notamment par la technique Biacore susmentionnée,
- le cas échéant, mise en présence des molécules sélectionnées à l'étape précédente avec des cellules exprimant des canaux calciques, et observation de l'éventuel effet des molécules sélectionnées sur la régulation de l'état d'inactivation des canaux calciques neuronaux impliqués dans la libération des neurotransmetteurs desdites cellules, notamment par mesures électrophysiologiques.

L'invention a également pour objet l'utilisation de fragments peptidiques correspondant à la boucle III-IV de la sous-unité α1, ou à une séquence peptidique dérivée, ou à une partie de cette boucle III-IV, ou de cellules transformées par des séquences nucléotidiques codant pour lesdits fragments, tels que définis ci-dessus, pour la mise en oeuvre de procédés de criblage de molécules régulant l'état d'inactivation des canaux calciques neuronaux impliqués dans la libération des neurotransmetteurs, telles que définies ci-dessus, notamment l'utilisation:
- de fragments peptidiques de la boucle III-IV de la sous-unité α1 des canaux calciques de mammifères, lesdits fragments :
   **(i).** correspondant à la sous-unité Cav2.1 des canaux calciques de cellules neuronales humaines correspondant à la séquence SEQ ID NO : 7 suivante :
      ITFQEQGDKMMEEYSLEKNERAClDFAISAKPLTRHMPQNKQSFQYRMWQFVVSP
   **(ii).** ou correspondant à une partie de ladite boucle III-IV.
- ou de cellules transformées :
   **(i).** avec la séquence nucléotidique codant pour la boucle III-IV de la sous-unité Cav2.1 des canaux calciques de cellules neuronales humaines, ladite séquence correspondant à la séquence SEQ ID NO : 6 suivante :
   **(ii).** ou avec la séquence nucléotidique codant pour la boucle III-IV de la sous-unité Cav2.1 des canaux calciques de cellules neuronales de lapin, ladite séquence correspondant à la séquence SEQ ID NO : 8 suivante :
pour la mise en oeuvre de procédés de criblage :
- de molécules restaurant le nombre de canaux calciques à la normale dans les membranes cellulaires où ce nombre a anormalement augmenté, à savoir du criblage de molécules susceptibles d'être utilisées dans le cadre du traitement de pathologies liées à une augmentation anormale du nombre de canaux calciques telle que l'hypertrophie cardiaque,
- et/ou de molécules réduisant le nombre de canaux calciques dans les membranes cellulaires, à savoir du criblage de molécules susceptibles d'être utilisées dans le cadre du traitement de pathologies contre lesquelles une diminution du nombre de canaux calciques à la membrane plasmique aurait un effet bénéfique, telle que l'épilepsie, l'hypertension, l'angine de poitrine, ou l'ischémie cérébrale,
- et/ou de molécules régulant l'état d'inactivation des canaux calciques neuronaux impliqués dans la libération des neurotransmetteurs, à savoir du criblage de molécules susceptibles d'être utilisées dans le cadre de la stimulation ou de l'inhibition de la communication neuronale, notamment dans le cadre du traitement de pathologies contre lesquelles une régulation de l'état d'inactivation des canaux calciques neuronaux impliqués dans la libération des neurotransmetteurs aurait un effet bénéfique, telles que les épilepsies, les ataxies, les migraines, la maladie de Parkinson, ou les ischémies cérébrales.

De préférence dans le cas de l'utilisation de fragments peptidiques de la sous-unité α1 ayant une taille supérieure à environ 5 acides aminés, lesdits fragments sont fusionnés ou non du côté N-terminal à une séquence peptidique transmembranaire, à savoir une séquence peptidique ayant pour effet de maintenir lesdits fragments peptidiques dans la membrane cellulaire, telle que la séquence transmembranaire de la chaîne a du récepteur CD8 humain contenue dans la séquence SEQ ID NO : 5 suivante :
LDFACDIYIWAPLAGTCGVLLLSLVITLYCNHR

En variante, l'invention concerne également l'utilisation susmentionnée de cellules transformées avec une séquence nucléotidique recombinante exogène codant pour une séquence peptidique transmembranaire telle que définie ci-dessus, cette dernière séquence étant située en amont de la séquence codant pour le fragment peptidique susmentionné de la sous-unité α1.

L'invention a également pour objet tout procédé de criblage de molécules régulant l'état d'inactivation des canaux calciques neuronaux impliqués dans la libération des neurotransmetteurs, telles que définies ci-dessus, caractérisé en ce qu'il comprend les étapes suivantes :
- mise en présence de fragments peptidiques de la sous-unité α1 tels que ci-dessus, avec des cellules exprimant des canaux calciques pendant un temps suffisant pour que l'état d'inactivation des canaux soit modifié, puis avec des molécules à tester,
- ou mise en présence de cellules transformées à l'aide de séquences nucléotidiques codant pour des fragments peptidiques de la sous-unité α1tels que définis ci-dessus, avec des molécules à tester,
- détection de l'effet des molécules testées sur l'état d'inactivation des canaux calciques neuronaux impliqués dans la libération des neurotransmetteurs, notamment par mesures électrophysiologiques.

Dans la présente invention, les Inventeurs utilisent les séquences peptidiques dérivées par mutation d'un ou plusieurs acides aminés des fragments peptidiques de la boucle III-IV de la sous-unité α1 des canaux calciques, la ou les mutations en question affectant des acides aminés essentiels dans le cadre de l'inactivation des canaux calciques présents à la surface membranaire, dans la mesure où leur mutation a pour effet de moduler l'activité des canaux calciques, pour la mise en oeuvre de procédés de criblage de molécules régulant l'état d'inactivation des canaux calciques neuronaux impliqués dans la libération des neurotransmetteurs, telles que définies ci-dessus.

Les Inventeurs utilisent plus particulièrement les séquences peptidiques comportant une ou plusieurs mutations ayant pour effet d'inactiver les canaux calciques, lesdites séquences peptidiques étant dérivées par mutation d'un au moins des acides aminés de la séquence peptidique correspondant à la séquence SEQ ID NO : 7.

Les Inventeurs utilisent encore plus particulièrement les séquences peptidiques comportant une ou plusieurs mutations ayant pour effet d'inactiver les canaux calciques, lesdites séquences peptidiques étant dérivées par mutation d'un au moins des acides aminés situés entre les positions 8 et 19 de la séquence peptidique correspondant à la séquence SEQ ID NO : 7, à savoir d'un au moins des acides aminés compris dans la séquence : DKMMEEYSLEKN.

L'invention concerne également tout procédé de criblage de molécules régulant l'état d'inactivation des canaux calciques neuronaux impliqués dans la libération des neurotransmetteurs, telles que définies ci-dessus, caractérisé en ce qu'il comprend les étapes suivantes :
- mise en présence de séquences peptidiques dérivées de la boucle III-IV de la sous-unité α1 tels que définies ci-dessus, avec des molécules à tester déjà sélectionnées pour leur capacité à se lier spécifiquement aux séquences peptidiques non mutées correspondant auxdites boucles III-IV,
- sélection des molécules susmentionnées se liant spécifiquement aux boucles III-IV et ne se liant pas aux séquences peptidiques dérivées susmentionnées, notamment selon la technique Biacore susmentionnée,
- le cas échéant, mise en présence des molécules sélectionnées à l'étape précédente avec des cellules exprimant des canaux calciques, et observation de l'éventuel effet des molécules sélectionnées sur la régulation de l'état d'inactivation des canaux calciques neuronaux impliqués dans la libération des neurotransmetteurs desdites cellules, notamment par mesures électrophysiologiques.

Les Inventeurs ont mis en évidence les séquences peptidiques suivantes :
- les séquences SEQ ID NO : 2 et SEQ ID NO : 4 dans lesquelles l'un au moins de Q en position 24, W en position 36, I en position 37, K en position 39, K en position 68, et K en position 69, est substitué par un acide aminé naturel ou non, notamment par une alanine,
- les séquences SEQ ID NO : 2 et SEQ ID NO : 4 dans lesquelles l'un au moins de R en position 28, R en position 63, et R en position 64, est substitué par un acide aminé naturel ou non, notamment par une alanine,
- les séquences SEQ ID NO : 2 et SEQ ID NO : 4 dans lesquelles l'un au moins de R en position 28, et E en position 29, est substitué par un acide aminé naturel ou non, notamment R28 est substitué par une alanine ou par E, et E29 est substitué par une alanine,
- la séquence SEQ ID NO : 15 suivante : QQIERELNGYMEWISKAE.

L'invention sera davantage illustrée à l'aide des résultats expérimentaux qui suivent:

### I- Démonstration de la diminution du nombre de canaux calciques à la surface membranaire des cellules chromaffines bovins et des cellules granulaires du cervelet de rat.

Les résultats des Inventeurs démontrent que l'expression de la boucle I-II de la sous-unité Cav2.1 dans les cellules chromaffines de bovins ou les cellules granulaires de cervelet induit une diminution notable des canaux calciques haut-seuils à la surface membranaire de ces cellules. Les mesures effectuées montrent que plus de 50% du courant calcique haut-seuil est diminué dans ces cellules suite à l'expression de cette séquence.

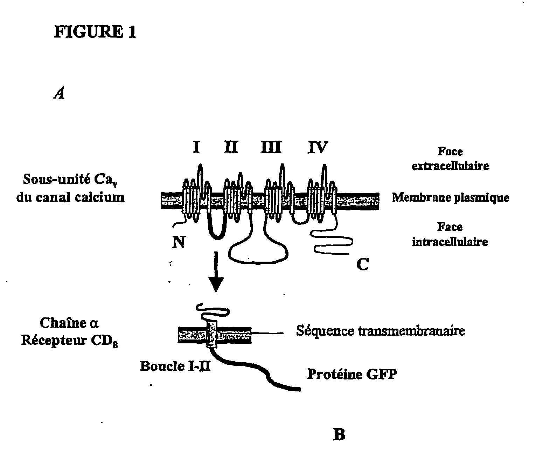

L'expression de la boucle I-II du canal Caᵥ2.1 est induite suite à une méthode de transfection cellulaire (grâce à un agent de transfection : lipofectamine, fugène, etc...). Afin d'avoir la capacité de fixer les sous-unités β endogènes à ces cellules, nous avons constaté que la capacité de cette séquence I-II à réduire l'expression à la membrane plasmique des canaux calciques haut-seuils endogènes est facilitée par son couplage à un segment transmembranaire. Les Inventeurs ont utilisé pour leurs expériences le segment transmembranaire de la chaine α du récepteur CD8 humain, couplé à la partie amino-terminale de la boucle I-II. Afin de faciliter la détection des cellules transfectées, la partie carboxy-terminale de la boucle I-II a été couplée à la protéine GFP (Green Fluorescent Protein) qui possède la propriété de fluorescence verte (facilement détectable en microscopie à fluorescence). La figure 1A illustre la construction utilisée pour ces expériences. En figure 1B, est illustrée la séquence en acide-aminés de la séquence transmembranaire de la chaine α du récepteur CD8.

Séquence transmembranaire de la chaîne α du récepteur CD8 humain (contenu dans) :
LDFACDIYIWAPLAGTCGVLLLSLVITLYCNHR

### II- Démonstration de l'importance des acides aminés Arg 387 et Glu 388 de la séquence Caᵥ2.1 de lapin dans l'inactivation de ce canal.

Les résultats expérimentaux obtenus démontrent que les acides aminés de la boucle I-II du canal calcique Caᵥ2.1 interviennent dans l'inactivation de ce canal. Deux modes de participation sont possibles :
1- En absence de sous-unité β, ces deux acides aminés sont impliqués dans une interaction moléculaire avec la boucle III-IV de la sous-unité Caᵥ2.1 (Figures 2 et 3). Si ces interactions sont interrompues, soit par mutagenèse des acides aminés Arg 387 ou Glu 388 (Figure 4), soit en exprimant la boucle III-IV (qui peut alors entrer en compétition avec l'interaction naturelle entre les boucles I-II et III-IV du canal Caᵥ2.1 ; Figure 5), l'inactivation du canal calcique est facilitée. Une inactivation facilitée représente, soit une transition accélérée de l'état ouvert du canal vers son état inactivé, soit une diminution du nombre de canaux activables à un potentiel membranaire donné de la cellule. Les méthodologies permettant la mesure des courants calciques et l'analyse de l'inactivation sont données en annexe A.
2- En présence de sous-unité β (la configuration normale du canal à la membrane plasmique), la charge électrique portée par les chaînes radicales de ces acides aminés (charge + pour Arg 387 et charge - pour Glu 388) joue un rôle dans l'inactivation de ce canal.

Les résultats démontrent que dans ces deux cas de figures, ces acides aminés peuvent être prises comme cible d'une intervention pharmacologique directe pour moduler l'inactivation des canaux calciques. La présence de ces acides aminés dans la plupart des canaux calciques neuronaux (Caᵥ2.1, Caᵥ2.2 et Caᵥ2.3), et chez la plupart des espèces, y compris chez l'homme suggère fortement que ces acides aminés interviennent également dans l'inactivation de ces canaux.

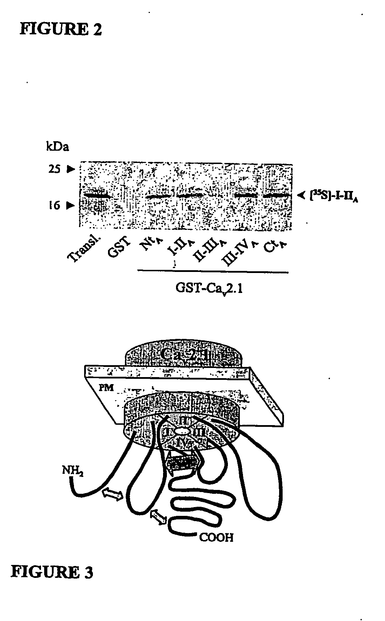

**Figure 2** **:** Fixation de la boucle I-II de Caᵥ2.1 sur différentes boucles intracellulaires.

Une fixation de I-II est évidente sur la boucle III-IV de Caᵥ2.1. Cette boucle III-IV a été exprimée et purifiée comme une protéine de fusion GST (Glutathion-S-Tranferase). La protéine I-II du canal a été traduite in vitro et marquée par un acide aminé radioactif (³⁵S-methionine) pour suivre sa fixation sur la protéine de fusion GST-III-IV.

**Figure 3** **:** Représentation schématique démontrant les interactions intramoléculaires entre les différentes boucles du canal Caᵥ2.1. La flèche verte illustre l'interaction entre la boucle I-II et la boucle III-IV de ce canal. PM = membrane plasmique.

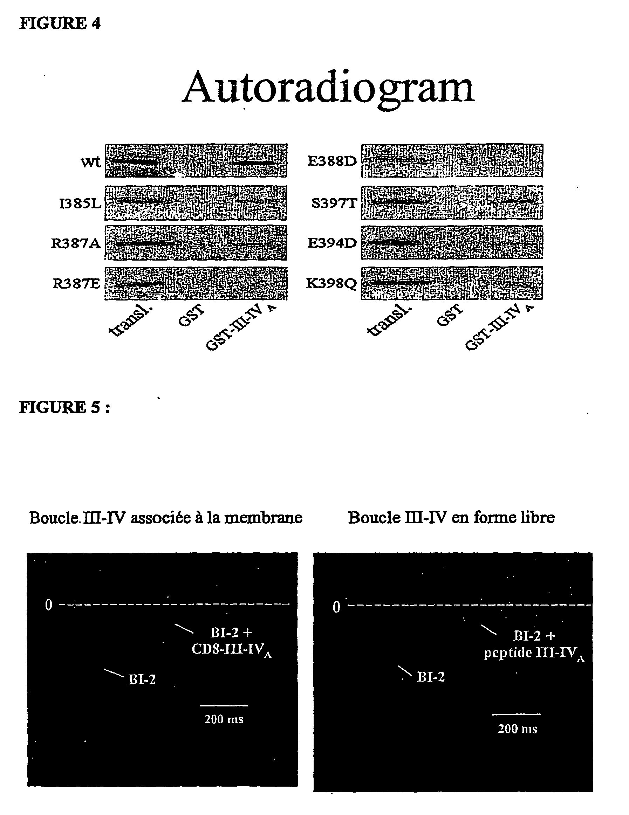

**Figure 4** **:** Démonstration de la perte de fixation de la boucle I-II du canal Cav2.1 sur la protéine de fusion GST-III-IV suite à la mutation des acides aminés Arg 387 ou Glu 388. Ces deux acides aminés sont donc indispensables à la fixation de la boucle I-II sur la boucle III-IV en absence de la sous-unités β du canal calcium.

**Figure 5** **:** L'expression de la boucle III-IV accélère la cinétique d'inactivation du canal Cav2.1. Cette accélération est similaire si la boucle III-IV exprimée est associée à la membrane plasmique (gauche, via un couplage en position amino-terminale avec la chaîne α du récepteur CD8 humain) ou est libre dans le cytoplasme (droite, injection d'un peptide représentant les 40 acides aminés carboxy-terminal de la boucle III-IV).

### III- Démonstration de l'importance de plusieurs acides aminés de la boucle I-II de la séquence Caᵥ2.1 de lapin dans la rétention de ce canal au niveau du réticulum endoplasmique. (Seulement information technique supplémentaire)

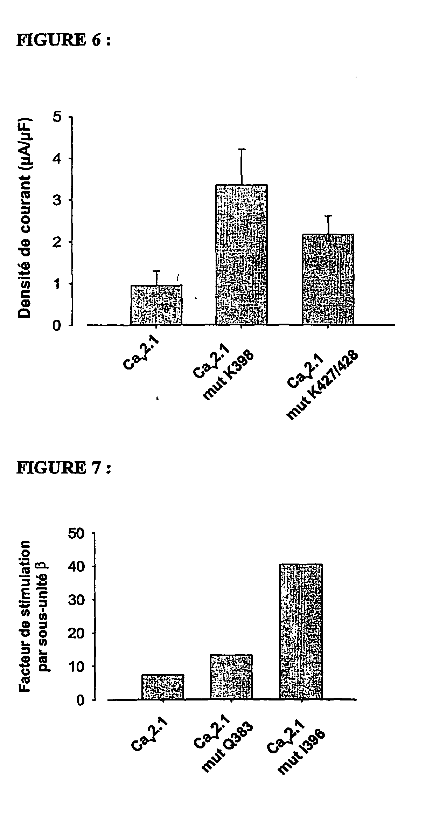

Les résultats de mutagenèse de la boucle I-II de la sous-unité Caᵥ2.1 et de l'expression dans l'ovocyte de Xénope des mutants correspondants démontrent qu'un certain nombre d'acide aminés de la boucle I-II peuvent être choisies comme cible pour augmenter l'adressage membranaire des canaux calciques haut-seuils. Nous pouvons classer ces acides aminés en deux catégories : (i) les acides aminés dont la fonction est indépendante de la sous-unité β des canaux calciques et dont la mutation induit une augmentation d'expression des canaux calciques à la surface de la membrane, et (ii) les acides aminés dont la mutation seule ne contribue pas à une expression de surface facilitée du canal Caᵥ2.1 lorsqu'il est exprimé seul, mais plutôt à une facilitation de l'action des sous-unités β. L'action desdites sous-unités β étant justement de faciliter l'expression des canaux calciques haut-seuils, l'expression à la surface cellulaire de l'ensemble du complexe Caᵥ2.1/β est également facilitée par la mutagenèse de ces résidus. L'importance des acides aminés β-indépendant est démontrée dans la figure 6, alors que celle des résidus β-dépendants est illustrée en figure 7. La figure 8 récapitule la position des acides aminés de la boucle I-II dont la mutation peut faciliter l'expression des canaux calciques.

**Figure 6** **:** Expression de sous-unités Caᵥ2.1 mutés augmentant l'expression du canal (en absence de sous-unité β). Mesure en densité de courants à la surface de la membrane plasmique des ovocytes de Xénopes. Voir annexe A ci-après pour la méthodologie d'expression et d'enregistrement des courants électriques.

**Figure 7****:** Expression de sous-unités Caᵥ2.1 mutés facilitant l'action de stimulation d'expression de la surface membranaire des canaux calciques par la sous-unité β. Voir annexe A pour la méthodologie d'expression et d'enregistrement des courants électriques.

Ci-après sont représentées les positions des acides aminés dont la mutation favorise l'expression à la surface de la membrane plasmique du canal Caᵥ2.1. En gras, acides aminés β-indépendant, et souligné, les acides aminés β-dépendants.

On peut aussi identifier la position des acides aminés dont la mutation entraîne une diminution de l'expression à la surface des cellules. Cette information est donnée à titre d'exemple ci-après.

Position des acides aminés dont la mutation entraîne une diminution notable d'expression du canal Caᵥ2.1 à la membrane (en présence et absence de sous-unité β).

Afin d'étendre la validité de nos résultats, nous avons aligné la séquence de la boucle I-II du canal Caᵥ2.1 de lapin à celle de la séquence I-II du canal Caᵥ2.1 humain. Les positions des acides aminés essentiels à l'ensemble des fonctions citées sont indiquées en gras ci-après.

Alignement des séquences de Caᵥ2.1 de lapin et humain. En gras, acide aminés essentiels. Souligné : les acides aminés différents entre les deux séquences.

### Annexe A

### Expression du canal Caᵥ2.1 dans l'ovocyte de Xénope, mesure électrophysiologique des courants calciques et analyse des propriétés d'inactivation du canal calcique.

### Expression

Les ovocytes de Xénopes sont prélevés aux stades V et VI à partir d'ovaires de grenouille *Xenopus laevis* d'afrique du sud et maintenus en solution saline classique (milieu de Barth). Les cellules sont traitées à la collagenase type IA (2 mg/ml) pendant deux heures et les membranes folliculaires sont éliminées manuellement. Les ovocytes sont maintenus en milieu DNOM (defined nutrient oocyte medium) durant 2 jours avant injection de cRNA codant pour le canal Cav2.1. L'injection de ce cRNA codant pour le canal sauvage ou muté est effectué à une concentration de 0.3 µg/µl. Dans les cas de coinjection d'autres cRNA (exemple : CD8-III-IV, III-IV, etc...), ces cRNA sont injectés à une concentration de 0.1 µg/µl. Les injections de peptide III-IV sont effectuées à une concentration finale intracellulaire de 10 µM. Dans tous les cas de figure, le volume d'injection n'excède pas 50 nl par ovocyte. Suite aux injections de cRNA, les cellules sont conservées à 16°C en milieu DNOM pendant au minimum 4-5 jours avant de poursuivre avec les enregistrements électrophysiologiques.

### Mesures électrophysiologiques

Nous avons appliqué la technique de voltage-clamp à deux electrodes pour les enregistrements de courants Ba²⁺ (le Ba²+ est plus perméable que le Ca²⁺ au travers des canaux calcium à haut-seuils et facilite nos analyses). Les enregistrements sont effectués avec un amplificateur GeneClamp de chez Axon Instruments (Foster City, CA). Le milieu d'enregistrement extracellulaire contient (en mM): Ba(OH)₂ 40, NaOH 50, KCI 3, HEPES 5, acide niflumique 1, pH 7.4 avec l'acide methane sulfonique. Les électrodes sont remplies avec (en mM) KCl 140, EGTA 10 et HEPES 10 (pH 7.2 avec du KOH) et ont des résistances électriques comprises entre 0.5 et 1 MΩ. Les enregistrements de courant électriques sont filtrés « on-line » à 2 kHz, le courant de fuite est soustrait par un protocole P/6, et échantillonné à 5-10 kHz. Les données sont analysées avec le suite de logiciels pCLAMP version 6.03 (Axon Instruments).

### Analyse des propriétés d'inactivation du canal Caᵥ2. 1

Afin d'établir des courbes d'inactivation en fonction du potentiel membranaire de cellule, les ovocytes sont depolarisés pendant au moins 30 secondes à une valeur de potentiel donnée (entre -100 et 10 mV ; potentiel de maintien), puis le courant calcique est déclenché par une dépolarisation cellulaire à +20 mV. Cette procédure est répétée plusieurs fois pour des valeurs de potentiel de maintien croissant (de 10 mV en 10 mV). L'amplitude maximale des courants Ba²⁺ ainsi obtenues pour chaque dépolarisation à +20 mV est comparée à l'amplitude du courant maximal obtenu par une dépolarisation à partir du potentiel de -100 mV vers +20 mV (valeur référence correspondant à 0% d'inactivation des canaux). L'inactivation des canaux calciques est aussi déclenchée par une dépolarisation maintenue à +20 mV (à partir d'une valeur de maintien de -100 mV). Le décours cinétique décroissant du courant illustre le processus d'inactivation des canaux calciques qui est déclenché par la dépolarisation à +20 mV. Pour comparer les différences en cinétiques d'inactivation, nous avons choisit le temps nécessaire à la demi-inactivation des canaux calciques comme valeur de référence.

## Revendications

1. Utilisation :
- de fragments peptidiques de la boucle III-IV de la sous-unité α1 des canaux calciques de mammifères, lesdits fragments :
**(i).** correspondant à la sous-unité Cav2.1 des canaux calciques de cellules neuronales humaines correspondant à la séquence SEQ ID NO : 7 suivante :
ITFQEQGDKMMEEYSLEKNERACIDFAISAKPLTRHMPQNKQSFQYRMWQFVVSP
**(ii).** ou de séquences peptidiques comportant une ou plusieurs mutations ayant pour effet d'inactiver les canaux calciques, lesdites séquences peptidiques étant dérivées par mutation d'un au moins des acides aminés **situés entre les positions 8 et 19** de la séquence peptidique correspondant à la séquence SEQ ID NO : 7, **à savoir d'un au moins des acides aminés compris dans la séquence : DKMMEEYSLEKN.**
**(iii).** ou correspondant à une partie de ladite boucle III-IV ou d'une séquence dérivée de cette dernière **ayant la propriété de ladite boucle III-IV de se lier à la boucle I-II.**
- ou de cellules transformées :
**(i).** avec la séquence nucléotidique codant pour la boucle III-IV de la sous-unité Cav2.1 des canaux calciques de cellules neuronales humaines, ladite séquence correspondant à la séquence SEQ ID NO : 6 suivante :
**(ii).** ou avec la séquence nucléotidique codant pour la boucle III-IV de la sous-unité Cav2.1 des canaux calciques de cellules neuronales de lapin, ladite séquence correspondant à la séquence SEQ ID NO : 8 suivante :
- de molécules restaurant le nombre de canaux calciques à la normale dans les membranes cellulaires où ce nombre a anormalement augmenté, à savoir du criblage de molécules susceptibles d'être utilisées dans le cadre du traitement de pathologies liées à une augmentation anormale du nombre de canaux calciques telle que l'hypertrophie cardiaque,
- et/ou de molécules réduisant le nombre de canaux calciques dans les membranes cellulaires, à savoir du criblage de molécules susceptibles d'être utilisées dans le cadre du traitement de pathologies contre lesquelles une diminution du nombre de canaux calciques à la membrane plasmique aurait un effet bénéfique, telle que l'épilepsie, l'hypertension, l'angine de poitrine, ou l'ischémie cérébrale,
- et/ou de molécules régulant l'état d'inactivation des canaux calciques neuronaux impliqués dans la libération des neurotransmetteurs, à savoir du criblage de molécules susceptibles d'être utilisées dans le cadre de la stimulation ou de l'inhibition de la communication neuronale, notamment dans le cadre du traitement de pathologies contre lesquelles une régulation de l'état d'inactivation des canaux calciques neuronaux impliqués dans la libération des neurotransmetteurs aurait un effet bénéfique, telles que les épilepsies, les ataxies, les migraines, la maladie de Parkinson, ou les ischémies cérébrales.

2. Utilisation de fragments peptidiques de la sous-unité α1 selon la revendication 1, lesdits fragments étant fusionnés du côté N-terminal à une séquence peptidique transmembranaire, à savoir une séquence peptidique ayant pour effet de maintenir lesdits fragments peptidiques dans la membrane cellulaire, telle que la séquence transmembranaire de la chaîne α du récepteur CD8 humain contenue dans la séquence SEQ ID NO : 5 suivante :
LDFACDIYIWAPLAGTCGVLLLSLVITLYCNHR
ou de cellules transformées avec une séquence nucléotidique recombinante exogène codant pour une séquence peptidique transmembranaire telle que définie ci-dessus, cette dernière séquence étant située en amont de la séquence codant pour le fragment peptidique susmentionné de la sous-unité α1.

3. Procédé de criblage de molécules régulant l'état d'inactivation des canaux calciques neuronaux impliqués dans la libération des neurotransmetteurs, telles que définies dans la revendication 1, **caractérisé en ce qu'**il comprend les étapes suivantes :
- mise en présence de fragments peptidiques de la sous-unité α1 tels que définis dans l'une des revendications précédentes, avec des cellules exprimant des canaux calciques pendant un temps suffisant pour que l'état d'inactivation des canaux soit modifié, puis avec des molécules à tester,
- ou mise en présence de cellules transformées à l'aide de séquences nucléotidiques codant pour des fragments peptidiques de la sous-unité α1 tels que définis dans la revendication 1, avec des molécules à tester,
- détection de l'effet des molécules testées sur l'état d'inactivation des canaux calciques neuronaux impliqués dans la libération des neurotransmetteurs.

4. Procédé de criblage de molécules régulant l'état d'inactivation des canaux calciques neuronaux impliqués dans la libération des neurotransmetteurs, telles que définies dans la revendication 1, **caractérisé en ce qu'**il comprend les étapes suivantes :
- mise en présence de séquences peptidiques dérivées de la boucle III-IV de la sous-unité α1 tels que définies dans l'une des revendications 1 ou 2, avec des molécules à tester déjà sélectionnées pour leur capacité à se lier spécifiquement aux séquences peptidiques non mutées correspondant auxdites boucles III-IV,
- sélection des molécules susmentionnées se liant spécifiquement aux boucles III-IV et ne se liant pas aux séquences peptidiques dérivées susmentionnées,
- le cas échéant, mise en présence des molécules sélectionnées à l'étape précédente avec des cellules exprimant des canaux calciques, et observation de l'éventuel effet des molécules sélectionnées sur la régulation de l'état d'inactivation des canaux calciques neuronaux impliqués dans la libération des neurotransmetteurs desdits cellules.

## Claims

1. Use :
- of the peptide fragments of the III-IV loop of a α1 sub-unit from the calcium channels of mammals, said fragments:
(i). corresponding to a Cav2.1 sub-unit of the calcium channels of human neuronal cells corresponding to the following sequence SEQ ID NO: 7:
ITFQEQGDKMMEEYSLEKNERACIDFAISAKPLTRHMPQNKQSFQYRMWQFVVSP
(ii). or the peptide sequences comprising one or more mutations having the effect of inactivating the calcium channels, said peptide sequences being derived by mutation of at least one of the amino acids situated between positions 8 and 19 of the peptide sequence corresponding to the sequence SEQ ID NO: 7, namely of at least one of the amino acids included in the sequence: DKMMEEYSLEKN,
(iii). or corresponding to a part of said III-IV loop, or of a sequence derived from the latter, having the the property of said III-IV loop of binding to a I-II loop.
- or of cells transformed:
(i) by the nucleotide sequence coding for a III-IV loop of a Cav2.1 sub-unit of the calcium channels of human neuronal cells, said sequence corresponding to the following sequence SEQ ID NO: 6:
(ii) or by the nucleotide sequence coding for a III-IV loop of a Cav2.1 sub-unit of the calcium channels of rabbit neuronal cells, said sequence corresponding to the following sequence SEQ ID NO: 8:
- of molecules restoring the number of calcium channels to normal in cell membranes where this number has abnormally increased, namely screening molecules capable of being used in the treatment of pathologies linked to an abnormal increase in the number of calcium channels such as cardiac hypertrophy,
- and/or of molecules reducing the number of calcium channels in the cell membranes, namely screening molecules capable of being used in the treatment of pathologies against which a reduction in the number of calcium channels in the plasma membrane would have a beneficial effect, such as epilepsy, hypertension, angina pectoris, or cerebral ischaemia,
- and/or molecules regulating the state of inactivation of the neuronal calcium channels involved in the release of neurotransmitters, namely screening molecules capable of being used in the stimulation or inhibition of neuronal communication, in particular in the treatment of pathologies against which a regulation of the state of inactivation of the neuronal calcium channels involved in the release of neurotransmitters would have a beneficial effect, such as epilepsy, ataxia, migraine, Parkinson's disease, or cerebral ischaemia.

2. Use of the peptide fragments of the α1 sub-unit according to claim 1, wherein said fragments are fused on the N-terminal side to a transmembrane peptide sequence, namely a peptide sequence having the effect of maintaining said peptide fragments in the cell membrane, such as the transmembrane sequence of the α chain of the human CD8 receptor contained in the following sequence SEQ ID NO: 5:
LDFACDIYIWAPLAGTCGVLLLSLVITLYCNHR
or of cells transformed with an exogenous recombinant nucleotide sequence coding for a transmembrane peptide sequence as defined above, this last sequence being situated upstream of the sequence coding for said peptide fragment of the α1 sub-unit.

3. Process for screening molecules regulating the state of inactivation of the neuronal calcium channels involved in the release of neurotransmitters as defined in claim 1,
- bringing together peptide sequences of the α1 sub-unit as defined in preceding claims, and cells expressing calcium channels for a time sufficient for the state of inactivation of the channels to be modified, then with the molecules to be tested,
- or bringing together cells transformed using nucleotide sequences coding for peptide fragments of the α1 sub-unit and the molecules to be tested,
- and detecting the effect of the molecules tested on the state of inactivation of the neuronal calcium channels involved in the release of neurotransmitters.

4. Process for screening molecules regulating the state of inactivation of the neuronal calcium channels involved in the release of neurotransmitters as defined in claim 1, **characterized in that** it comprises the following steps:
- bringing together peptide sequences derived from the III-IV loop of the α1 sub-unit according to claim 1 or 2, and the molecules to be tested already selected for their ability to bind specifically to the non-mutated peptide sequences corresponding to said III-IV loops,
- selecting said molecules binding specifically to the III-IV loops and not binding to said derived peptide sequences,
- if appropriate, bringing together the molecules selected in the previous stage and cells expressing calcium channels, and observing any effect of the molecules on the regulation of the state of inactivation of the neuronal calcium channels involved in the release of neurotransmitters from said cells.

## Patentansprüche

1. Verwendung:
- von Peptidfragmenten der III-IV-Schleife der α1-Untereinheit der Calciumkanäle von Säugern, wobei die Fragmente:
(i) der Cav2.1-Untereinheit der Calciumkanäle von humanen Nervenzellen entsprechen, die der folgenden Sequenz SEQ ID NO: 7 entspricht:
ITFQEQGDKMMEEYSLEKNERACIDFAISAKPLTRHMPQNKQSF QYRMWQFVVSP
(ii) oder Peptidsequenzen, die eine oder mehrere Mutationen umfassen, deren Wirkung es ist, die Calciumkanäle zu deaktivieren, wobei die Peptidsequenzen abgeleitet sind durch Mutation mindestens einer der Aminosäuren, die zwischen den Positionen 8 und 19 der Peptidsequenz liegen, die der Sequenz SEQ ID NO: 7 entspricht, nämlich mindestens einer der Aminosäuren, die in der Sequenz:
DKMMEEYSLEKN enthalten sind,
(iii)oder einem Teil der III-IV-Schleife einer Sequenz entsprechen, die von dieser letztgenannten abgeleitet ist, die die Eigenschaft der III-IV-Schleife hat, sich mit der I-II-Schleife zu verbinden.
- oder transformierten Zellen:
(i) mit der Nucleotidsequenz, die für die III-IV-Schleife der Cav2.1-Untereinheit der Calciumkanäle von humanen Nervenzellen kodiert, wobei die Sequenz der folgenden Sequenz SEQ ID NO: 6 entspricht:
(ii) oder mit der Nucleotidsequenz, die für die III-IV-Schleife der Cav2.1-Untereinheit der Calciumkanäle von Nervenzellen des Kaninchens kodiert, wobei die Sequenz der folgenden Sequenz SEQ ID NO: 8 entspricht: zur Durchführung von Screeningverfahren:
- von Molekülen, die die Anzahl der Calciumkanäle in den Zellmembranen, in denen diese Anzahl abnorm erhöht ist, wieder auf den Normalwert bringen, nämlich dem Screenen von Molekülen, die im Rahmen der Behandlung von Pathologien verwendet werden können, die mit einer abormen Erhöhung der Anzahl der Calciumkanäle verbunden sind, wie etwa der Herzhypertrophie,
- und/oder von Molekülen, die die Anzahl der Calciumkanäle in den Zellmembranen reduzieren, nämlich das Screenen von Molekülen, die im Rahmen der Behandlung von Pathologien verwendet werden können, gegen die eine Verringerung der Anzahl der Calciumkanäle für die Plasmamembran eine positive Wirkung hätte, wie etwa Epilepsie, Bluthochdruck, Angina pectoris oder zerebrale Ischämie,
- und/oder von Molekülen, die den Inaktivierungszustand der neuronalen Calciumkanäle regulieren, die an der Freisetzung der Neurotransmitter beteiligt sind, nämlich das Screenen von Molekülen, die im Rahmen der Stimulation oder der Inhibition der neuronalen Kommunikation verwendet werden können, insbesondere im Rahmen der Behandlung von Pathologien, auf die eine Regulation des Inaktivierungszustands der neuronalen Calciumkanäle, die an der Freisetzung der Neurotransmitter beteiligt sind, eine positive Wirkung hätte, wie etwa Epilepsien, Ataxien, Migränen, Parkinson-Krankheit oder zerebrale Ischämien.

2. Verwendung von Peptidfragmenten der α1-Untereinheit nach Anspruch 1, wobei die Fragmente N-terminal mit einer Transmembran-Peptidsequenz fusioniert sind, nämlich einer Peptidsequenz, deren Wirkung es ist, die Peptidfragmente in der Zellmembran zu erhalten, wie etwa die Transmembransequenz der α-Kette des humanen CD8-Rezeptors, die in der folgenden Sequenz SEQ ID NO: 5 enthalten ist:
LDFACDIYIWAPLAGTCGVLLLSLVITLYCNHR
oder von Zellen, die mit einer exogenen rekombinanten Nucleotidsequenz transformiert sind, die für eine Transmembran-Peptidsequenz kodiert, wie oben definiert, wobei letztgenannte Sequenz stromaufwärts der Sequenz liegt, die für das oben genannte Peptidfragment der α1-Untereinheit kodiert.

3. Verfahren zum Screenen von Molekülen, die den Inaktivierungszustand der neuronalen Calciumkanäle regulieren, die an der Freisetzung von Neurotransmittern beteiligt sind, wie in Anspruch 1 definiert, **dadurch gekennzeichnet, dass** es die folgenden Schritte umfasst:
- Inkontaktbringen von Peptidfragmenten der α1-Untereinheit, wie in einem der vorhergehenden Ansprüche definiert, mit Zellen, die Calciumkanäle während eines Zeitraums exprimieren, der ausreicht, um den Inaktivierungszustand der Kanäle zu modifizieren, dann mit zu testenden Molekülen,
- oder Inkontaktbringen von Zellen, die mit Hilfe von Nucleotidsequenzen transformiert wurden, die für Peptidfragmente der α1-Untereinheit kodieren, wie in Anspruch 1 definiert, mit zu testenden Molekülen,
- Nachweis der Wirkung der getesteten Moleküle auf den Inaktivierungszustand der neuronalen Calciumkanäle, die an der Freisetzung der Neurotransmitter beteiligt sind.

4. Verfahren zum Screenen von Molekülen, die den Inaktivierungszustand der neuronalen Calciumkanäle regulieren, die an der Freisetzung von Neurotransmittern beteiligt sind, wie in Anspruch 1 definiert, **dadurch gekennzeichnet, dass** es die folgenden Schritte umfasst:
- Inkontaktbringen von Peptidsequenzen, die von der III-IV-Schleife der α1-Untereinheit abgeleitet sind, wie in einem der Ansprüche 1 oder 2 definiert, mit zu testenden Molekülen, die bereits auf ihre Fähigkeit selektiert wurden, spezifisch an die mutierten Peptidsequenzen zu binden, die den III-IV-Schleifen entsprechen,
- Selektion der oben genannten Moleküle, die spezifisch an die III-IV-Schleifen binden und die nicht an die oben genannten abgeleiteten Peptidsequenzen binden,
- gegebenenfalls Inkontaktbringen der im vorhergehenden Schritt selektierten Moleküle mit Zellen, die Calciumkanäle exprimieren, und Beobachtung der möglichen Wirkung der selektierten Moleküle auf die Regulation des Inaktivierungszustands der neuronalen Calciumkanäle, die an der Freisetzung der Neurotransmitter dieser Zellen beteiligt sind.
